(19) Europäisches Patentamt
European Patent Office
Office européen des brevets

(11) Publication number: **0 110 068 A2**

# (12) EUROPEAN PATENT APPLICATION

(21) Application number: 83110096.1

(22) Date of filing: 10.10.83

(51) Int. Cl.³: A 61 K 6/08, C 07 C 69/88

(30) Priority: 01.11.82 US 438296

(43) Date of publication of application: 13.06.84 Bulletin 84/24

(84) Designated Contracting States: AT CH DE FR GB IT LI NL SE

(71) Applicant: DENTSPLY INTERNATIONAL, INC., 570 West College Avenue, York Pennsylvania 17405 (US)

(72) Inventor: Wang, Wu Lan, 45 S. Horseshoe Drive, Milford, DE 19963 (US)
Inventor: Dougherty, Emery W., 451 Hunting Park Lane, York, PA 17402 (US)
Inventor: Smith, Roy L., 505 Caulk Roak, Milford, DE 19963 (US)

(74) Representative: Wächtershäuser, Günter, Dr., Tal 29, D-8000 München 2 (DE)

## (54) Hydrophobic dental cavity liners.

(57) Dental cavity lining compositions are provided which are substantially hydrophobic while retaining the ability to stimulate the production of secondary dentin by vital, pulpal tissues. According to a preferred embodiment, metal oxide or hydroxide-containing compositions are provided which comprise an amount sufficient to impart substantial hydrophobicity to the compositions of a fluorinated polymeric hydrocarbon. According to another preferred embodiment, the fluorinated polymeric hydrocarbon comprises polytetrafluoroethylene. The neopentyl diesters of a salicylic acid are described as being superior hardenable species for inclusion in such dental cavity linings.

EP 0 110 068 A2

HYDROPHOBIC DENTAL CAVITY LINERS

BACKGROUND OF THE INVENTION

This invention is directed to novel hydrophobic cavity liners for use in dentistry. In general, it has been known to prepare dental cavities for filling or restoration by applying thereto a dental cavity liner. Accordingly, a tooth in need of repair is excavated to remove decayed material and detritus. The exposed, internal portion of the tooth may then be lined with a dental cavity liner, etched with inorganic acid such as 37% phosphoric acid, and filled either with the traditional amalgam or with one of the modern, resin-based dental restorative compositions.

As will be appreciated by those skilled in the art, the function of a dental cavity liner is thought to be two fold. First, it serves as an insulative barrier for pulpal tissue against the attack of acid from certain filling materials; it neutralizes inorganic acids found in such filling materials and acts as a protective barrier against unpleasant stimuli. Additionally, when the cavity liner comprises certain metal oxides or hydroxides such as calcium oxide, zinc oxide, calcium hydroxide and certain others, the formation of secondary dentin is believed to be stimulated. This latter process is to be preferred in that it serves to revivify the tooth and to improve the likelihood of successful retention of the dental restoration. Such dental cavity liners have been disclosed in, for example, U.S. Patents 3,047,408-Dougherty, 3,266,147-Goldman, 3,390,456-DeLeva, 3,509,089-Dougherty, and 4,240,832-Jandourek. Metal oxide and hydroxide based cavity liners have also been extensively marketed. Dycal®, a product of the L.D. Caulk division of Dentsply International Inc., has been widely employed for the protection of pulpal tissues during tooth restoration.

Previously, it has been thought necessary to provide dental cavity lining materials which are water sorptive to

facilitate the elution of calcium hydroxide or other metal hydroxide or oxide from the cavity lining composition so that stimulation of secondary dentin could occur. This consideration has resulted in the preparation of dental lining compositions which are relatively unstable to water and which may, accordingly, be invaded by aqueous fluids and solutions.

The acid stability of present metal oxide and hydroxide based cavity lining materials is not as great as is desired; improvement of such stability has long been needed. Thus, contacting such cavity linings in prepared teeth with strong acid, such as 37% phosphoric acid, causes a rapid slaking of the liner through reaction between the acid and the metal oxide or hydroxide binder. Such slaking is believed to be detremental and is to be avoided. In addition, present cavity lining materials are relatively weak physically, especially after long term exposure to aqueous media. Stronger materials are greatly desired. Materials for cavity lining which allow less invasion of pulpal spaces by aqueous fluids are also greatly desired. See in this regard, Dentist's Desk Reference, American Dental Association, 1st ed. (1981) pg. 87.

U.S. Patent 3,469,317-Jarby et. al. relates to the filling of dental cavities by placing into a prepared cavity a water insoluble, pressure moldable synthetic resin, e.g. polytetrafluoroethylene, in finely divided form and then mechanically working and compacting the same into a solidified mass.

U.S. Patent 4,197,234-Temin discloses the addition of polytetrafluoroethylene in powder form to a conventional inert organic filler for dental restorative composition use. The combined filler is used in conjunction with a liquid polymerizable binder.

U.S. Patent 4,292,029-Craig et al relates to hydrophobic composite restorative materials which may include particulate fluorine-containing polymers in the binder system.

None of the foregoing patents or publications anticipates or renders obvious the novel hydrophobic, acid

resistant dental cavity lining compositions of the present invention.

## SUMMARY OF THE INVENTION

Novel dental cavity lining materials have now been discovered which are hydrophobic in nature. Such materials have been developed which are more stable toward water and aqueous acid than are prior compositions. The integrity and strength of the lining materials is greatly improved as well. Invasion of the pulpal spaces of filled teeth by aqueous fluids and solutions with the threat of reinfection is minimized through use of the dental lining compositions of the present invention. Moreover, it has been surprisingly discovered that the use of hydrophobic dental lining materials in accordance with the present invention further comprising calcium oxide, calcium hydroxide, zinc oxide, or other metal hydroxides or oxides provides the ability to stimulate the production of secondary dentin even though water sorptivity has been greatly diminished. Metal oxide or hydroxide containing dental cavity liners comprising an amount of a fluorinated polymeric hydrocarbon resin sufficient to impart hydrophobicity to the dental lining composition have now been prepared. It has also been discovered to provide novel polymerizable ester formulations comprising a diester of a salicylic acid and a neopentyl glycol to further comprise the dental lining materials in combination with fluorinated polymeric hydrocarbons. Methods for the improvement of resinous salicylate ester compositions in accordance with the present invention have been discovered whereby such resins are treated with inorganic acid at elevated temperatures to minimize agglomerization of such esters.

## OBJECTS OF THE INVENTION

It is an object of this invention to provide hydrophobic dental lining compositions.

It is another object to provide dental lining compositions having a diminished water solubility and a greater water and acid stability than previous materials.

It is a further object of this invention to provide dental lining compositions which are substantially hydrophobic while retaining the ability to stimulate the production of dentin by pulpal tissues.

A still further object is to provide dental cavity linings which minimize invasion of pulpal spaces by aqueous fluids and which diminish the threat of infection in those spaces.

Yet another object is to provide dental lining compositions comprising an amount of a fluorinated polymeric hydrocarbon which is sufficient to provide substantial hydrophobicity to the composition.

Another object of the invention is directed to the formulation of resin based systems for inclusion in the lining materials of this invention.

Another object is to provide diesters of a salicylic acid and a neopentyl glycol which are hardenable in the presence of an alkaline earth cation for use in dental cavity lining compositions.

A further object is to minimize agglomerization of neopentyl disalicylates by treating them with inorganic aqueous acid at elevated temperatures. These and other objects shall become apparent from a review of the present specification.

## DETAILED DESCRIPTION OF THE INVENTION

It has now been surprisingly discovered that metal oxide or hydroxide-containing dental cavity liners may be formulated so as to be substantially hydrophobic while retaining the ability to stimulate the production of secondary dentin in a repaired tooth. It is now possible to provide dental cavity lining materials which have improved stengths, improved resistence to attack by aqueous media, and greater resistance to aqueous acidic degradation. Moreover, improved sealing of

vital tissues against invasion by aqueous fluids and subsequent reinfection is provided through the employment of one or more embodiments of the present invention.

In general, the dental cavity liners of the present invention comprise metal oxides or hydroxides together with amounts sufficient to impart substantial hydrophobicity to the composition of one or more fluorinated polymeric hydrocarbons.

Those skilled in the art will appreciate that a wide variety of metal oxides or hydroxides may be suitable for use in the practice of the present invention. Thus, calcium hydroxide, calcium oxide, zinc oxide, and many other such materials may be employed. Both the calcium and zinc species work well, are inexpensive and, accordingly, are preferred. An amount of metal oxide or hydroxide is selected which is sufficient to stimulate the production of secondary dentin by the vital tissues of a tooth. In general, amounts of metal oxide or hydroxide of from about 10% to about 60% by weight of the lining composition are preferably selected. Mixtures of oxides and hydroxides may be employed if desired.

The fluorinated polymeric hydrocarbons which may be selected for employment in the embodiments of the present invention may be any of a wide variety of such polymers. Thus, polytetrafluoroethylene, polyvinylidene fluoride, polychlorotrifluoroethylene and many others, including mixtures, may be used. It is preferred that the fluorinated polymeric hydrocarbon be a solid at room temperature and that it be employed in finely comminuted or divided form. In general, a dispersion of the fluorinated polymer in the lining composition is desired.

An amount of fluorinated polymeric hydrocarbon is included in the dental lining compositions of the present invention which is sufficient to impart substantial hydrophobicity to the composition. In this regard, it is desired that the lining composition be substantially less soluble in water and aqueous acid than are presently available compositions. Accordingly, such materials are preferably formulated which have a 37°C 24 hour water solubility of less than

about 5% over the first 24 hours. Even more preferred are compositions which have water solubilities of less than about 3% in 24 hours.

It is also desired that the compositions be formulated so as to be relatively stable toward aqueous acid, especially phosphoric acid. In this regard, compositions having solubilities of less than about 3% in 36% phosphoric acid after 10 minutes are preferred. It is even more preferred that acid solubilities less than about 2% be demonstrated. While the foregoing water and acid stability tests are indicative of hydrophobicity in accordance with this invention, another indication of hydrophobicity is available as well. Thus, an additional, but less reliable, method for the evaluation of hydrophobicity comprises an evaluation of water contact angle. This well-known procedure produces a numerical value for the contact interface between water and the surface of a material in question. High values such as above 30° and preferably above about 35° imply greater hydrophobicity. This evaluation is subject to certain errors, however, such as when the surfaces of materials to be compared have different microporosities. Notwithstanding this shortcoming, contact angle evaluation is a useful screening procedure for evaluation of hydrophobicity.

In accordance with the preferred practice of the present invention, compostions are formulated having, at once, large water contact angles, low water solubility and low aqueous acid solubility as described above. In general, a fluorinated polymeric hydrocarbon is included in the dental lining compositions of this invention in an amount of from about 2% to about 15% by weight of the total composition. It is even more preferred to employ from about 4% to about 10% of such fluorinated polymeric hydrocarbon in these compositions. Those skilled in the art will appreciate that the amounts of fluorinated polymeric hydrocarbon which are preferred for employment at any given composition will depend upon the chemical structure, molecular weight, and degree of fluorination of a particular polymer selected together with the properties of the remaining materials of the lining

compositions. It is believed to be within the skills of those ordinarily skilled in the art to select an amount of fluorinated hydrocarbon which is sufficient to impart substantial hydrophobicity for any particular lining composition in view of the foregoing guidelines.

In accordance with certain preferred embodiments, the lining compositions of the present invention will comprise materials in addition to the metal oxides or hydroxides and the fluorinated hydrocarbons. Thus, such materials generally may comprise radiopaqueing agents such as barium sulfate, titanium dioxide, calcium tungstate, and others. Amounts of such radiopaqueing materials are employed which are sufficient to impart radiopacity to the lining compositions when in use, generally from about 1% to about 5% by weight of the composition.

It is preferred to employ organic resin-based materials in the dental lining compositions of the present invention to impart settability and strength thereto. Thus, for example, any of those resinous materials disclosed in U.S. Patent 3,509,089-Dougherty, which is incorporated herein by reference, may be employed in the lining compositions in accordance with certain preferred embodiments of this invention. A wide variety of polymeric materials may be so employed. Thus, acrylates, (methacrylates, etc.), urethanes, vinyls, polyamides, polyesters, sulfonamides, and many other such species may be employed. Such materials are preferably provided with autocatalytic activity so that the same may be polymerized upon the mixing of separate resin and catalyst portions.

Certain dental lining materials known to the art, especially Dycal®, a product of the L.D. Caulk division of Dentsply International Inc., comprise phenolic compositions hardenable through exposure to a divalent metal hydroxide, especially an alkaline earth hyroxide, notably calcium hydroxide. See in this regard U.S. Patent 3,047,408,-Dougherty, which is incorporated herein by reference, for an explanation of this type of material. It has now been found that certain derivatives of salicylic acid are especially and surprisingly useful for inclusion as hardenable species in dental cavity lining compositions.

It has been discovered that the diesters of salicylic acid or closely related species with neopentyl glycol or closely related 2,2-dialkyl 1,3-dihydroxy propane, especially neopentyl glycol itself may be employed in the formulation of dental cavity lining compositions in accordance with the present invention to yield lining materials having improved strength over previously known phenolic materials.

The salicylic diesters which have been found to be preferred include the diesters of salicylic acid, 2-hydroxybenzoic acid, and related species herein referred to as a salicylic acid species. Thus the benzene ring of salicylic acid may have one or more substituents such as alkyl, halogen or the like as long as the phenolate salt hardening process described in U.S. Patent 3,047,408 is not substantially interfered with. The most preferred structural moiety for use is salicylic acid itself.

The 2,2-dialkyl 1,3-dihydroxy propanes useful in forming the diesters in accordance with the present invention may be represented by formula I:

I. 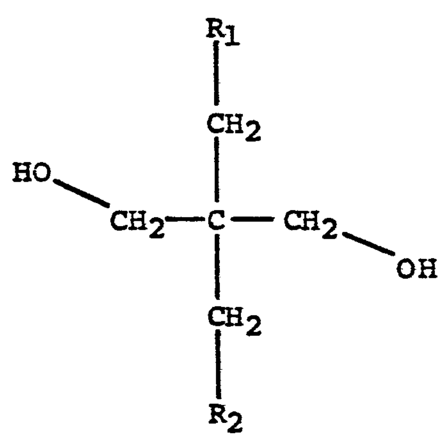

wherein $R_1$ and $R_2$ may be the same or different and are H or alkyl groups having from about 1 to about 3 carbon atoms. Most preferred among these glycols are neopentyl glycol itself ($R_1 = R_2 = H$) and 2,2-diethyl 1,3-propane diol ($R_1 = R_2 = CH_3$).

The diesters in accordance with this invention are preferably formulated by an alkoxide mediated transesterification. Thus, a salicylic ester such as methyl salicylate is combined with a 2,2-dialkyl 1,3-dihydroxy propane such as neopentyl glycol in a stoichiometry somewhat greater than about 2:1 (salicylate in excess). The blend is treated with, for example, sodium methoxide and heated to form the diester with concomitant removal of overhead products.

Diesters of 2,2-dialkyl 1,3-dihyroxy propanes with salicylates as described hereinabove tend to agglomerate upon formation. It has now been found that this agglomeration may be reduced or eliminated by heating the ester with aqueous inorganic acid subsequent to formulation of the esters.

The diesters of this invention are included in the dental cavity lining compositions of this invention in amounts sufficient to provide hardenability of such compositions upon their compounding with a divalent metal hydroxide especially an alkaline earth hydroxide. The hydroxide, such as the preferred calcium hydroxide, is provided in sufficient quantities as to furnish such hardenability. Preferably an excess of hydroxide is provided to contribute to improved revivification of pulpal tissues as discussed herein above.

The present compositions may also comprise pigments, fillers, viscosity modifiers, thixotropicity modifiers, and many other materials known to those skilled in the art. Thus, viscosity may be modified through the inclusion of fumed silica or other materials in such compositions. The addition of silica may also contribute to the hydrophobicity of the present compositions. The employment of certain pigments such as titanium dioxide and others may also serve to modify the viscosity or thixotropicity of the present compositions and also, incidentally, to modify the hydrophobicity thereof. It is envisioned that any of the foregoing materials together with numerous others known to those skilled in the art for inclusion in dental cavity liners may be employed in the practice of this invention.

It is also possible to formulate dental cavity liners in accordance with the present invention which are suspended in a carrier medium. Thus, aqueous or preferably organic solvents may be employed for diminishing the viscosity of the present materials or to effect "thinning" thereof. The solvent or carrier medium may then be removed through evaporation or otherwise to provide a layer of cavity liner. According to one embodiment, the carrier agent or solvent for the dental cavity liner comprises a polymerizable monomer such as an acrylic or methacrylic monomer. Rather than remove the monomer through evaporation, the same may be polymerized either through contact with a catalytic agent or through exposure to actinic light to result in polymerization and hardening of the monomer-dispersed cavity lining composition.

The employment of the dental cavity liner in accordance with the present invention is similar to the employment of certain prior cavity lining materials and is believed to be well understood to those skilled in the art. Thus, U.S. Patents 3,509,089 and 3,047,408-Dougherty, which have been incorporated herein by reference, disclose certain preferred means for such employment. The means for employment of Dycal®, a product of the L.D. Caulk Division of Dentsply International Inc., may also be employed for the application of the dental cavity liners of this invention Accordingly, it is necessary only to apply the cavity lining compositions of the present invention to an excavated tooth and to allow the same to harden, firm up, dry, or set. In accordance with a preferred embodiment, this hardening is accomplished through the reaction of a metallic hydroxide with certain salicylate esters. Hardening may also be accomplished through autocatalytic reactions or through photolyzation of polymerizable or crosslinkable species. Alternatively, evaporation of solvent or carrier media or the polymerization thereof may be employed to effect or contribute to hardening of the present compositions.

The following examples are intended as illustrative only and are not to be construed as limiting.

Example 1:

A two-component dental lining composition is formulated in accordance with the following procedure:

the diester of salicylic acid and neopentyl glycol is prepared by blending together 11 kg methyl salicylate (Crompton & Knowles) and 3.43 kg neopentyl glycol (Badische Corp.) at 40°C with mechanical stirring. Sodium methoxide, 191 grams, (Harshaw Co.) is then added while excluding moisture. The mixture is stirred further and heated to about 100°C over a period of about 1 hour. The blend is heated further while removing a distillate at pot temperatures of from about 100°C to about 200°C over a period of about 6 hours. About 2.5 liters of distillate is removed; the product ester bottoms become reddish brown. The material is cooled under $N_2$. Optimally, the ester may be treated with acid as follows. To the ester at about 70°C is added about 410 ml of about 19% HCl in water with stirring. The acidified solution is heated while blowing with nitrogen and about 400 ml of distillate collected. The bottoms temperature reaches about 200°C. The ester is then allowed to cool under nitrogen and decanted from any precipitate.

A base formulation is formed by combining the following:

Resin Base:

| | |
|---|---|
| diester of salicylic acid and neopentyl glycol | 52.0% |
| barium sulfate | 26.6 |
| calcium tungstenate | 5.4 |
| Polylube J-12 - polytetrafluoroethylene (Custom Compounding Inc.) | 13.8 |
| fumed silica (Aerosil R-972, Degussa) | 0.5 |
| fumed silica (Aerosil 200, Degussa) | 1.5 |
| pigment | 0.3 |
| | 100.1% |

A can mixer was charged with the diester. The remaining materials were tumbled together for 1 hour, sieved through a No. 10 screen, and tumbled for a further hour. The

tumbled blend was added to the diester slowly, with agitation to allow dispersal. After mixing for one further hour, the material was passed through a 3-roll mill having 0.0015 in. roll spacings. The material was returned to the can mixer where from about 0.1 to about 0.5% by weight of water was added. The amount of water may be varied to vary the setting time of the resulting formulations. The resin base was ready for use following a final pass through the 3-roll mill.

A catalyst is formulated from a blend of the following:

<u>Catalyst:</u>

| | |
|---|---|
| Santisizer 8® (Monsanto Acyl toluene sulfonamide) | 34.6% |
| calcium hydroxide | 50.6 |
| zinc oxide | 9.3 |
| zinc stearate | 0.3 |
| titanium dioxide | 4.7 |
| pigment | 0.6 |
| | 100.1% |

The sulfonamide was charged to a can mixer and agitated. The remaining components were slowly added in the order listed and the mixture blended for 45 minutes. The blend was twice passed through the 3-roll mill. The material was then placed in a covered candy kettle and mixed for 16 hours under a stream of $CO_2$. The carbonated mixture was replaced in the can mixer where from about 0.1 to about 0.5% by weight of water was incorporated. As before, the water content may be varied to vary the setting time. A final pass through the 3-roll mill placed the catalyst blend in condition for use.

Immediately prior to use, the resin base and catalyst are blended manually in a 1:1 ratio by volume until visibly uniform, about 15-20 seconds. The cavity liner is applied as a layer of about 1 mm to the surface of an excavation in an extracted tooth, covering all exposed, viable surfaces. The liner was applied directly to the pulp as a pulp capping agent and to the adjacent areas of dentin. The material begins to set-up or harden almost immediately. Application of the liner may be followed by application of a restorative

resin or amalgam to provide a tooth reconstruction wherein pulpal tissues are well insulated from attack by the aqueous oral environment. Good stimulation of the production of secondary dentin is also evidenced by the employment of this material.

Example 2:

Identical quantities of commercially available Dycal®, a product of the L.D. Caulk Division of Dentsply International Inc.; Life™, a product of the Kerr Company; Renew™, a product of the S.S. White Co.; and Procal™, a product of the 3M Co. together with the lining composition in accordance with Example 1 were formulated according to manufacturer's directions and allowed to harden over a period of about 2 hours. Each of the foregoing samples were immersed in 37°C water for a period of 24 hours and the amount of weight loss during that time calculated. Separate samples were immersed in 36% phosphoric acid for 10 minutes at room temperature and the amount of weight loss similarly calculated. It is desirable for dental cavity lining materials to exhibit low weight loss during each of those two tests. The data are as follows:

| | 24 hour water | 10 minute acid |
|---|---|---|
| Example 1 | 2.3% | 1.9% |
| Dycal® | 5.9% | 5.7% |
| Life™ | 8.1% | 3.0% |
| Renew™ | 1.0% | 13.3% |
| Procal™ | 8.4% | 27.8% |

Example 3

The contact angles of a drop of distilled water on the surface of the materials described in Example 2 were determined. Each of the materials was prepared in accordance with manufacturers' directions and formed so as to have a flat surface. Uniform drops of distilled water were placed upon each surface and the contact angle at the water-surface boundary measured with a Gaertner goniometer. Five

separate drops were placed upon five different locations on each surface and the individual values averaged. While surface porosity appears to distort the data, especially for Procal™, a view as to relative hydrophobicity could be obtained. The data follow; a higher angle implies greater hydrophobicity.

| | |
|---|---|
| Example 1 | 39° |
| Dycal® | 28° |
| Life™ | 29° |
| Renew™ | 18° |
| Procal™ | 34° |

Example 4

The improvement in strength realized by employment of neopentyl disalicylate esters in dental cavity lining compositions in accordance with the present invention may be demonstrated. A resin base was formulated which was similar in essence to that of Example 1 but with the substitution of the ester presently used in Dycal®, a product of the L.D. Caulk division of Dentsply International Inc.

| | |
|---|---|
| diester of salicylic acid and 1,3-butylene glycol | 52.1% |
| barium sulfate | 26.5 |
| calcium tungstenate | 5.4 |
| Polylube J-12 - polytetrafluoroethylene (Custom Compounding Inc.) | 13.8 |
| fumed silica (Aerosil R-972, Degussa) | 0.5 |
| fumed silica (Aerosil 200, Degussa) | 1.5 |
| pigment | 0.3 |
| | 100.1% |

This material was suitable for use with the catalyst of Example 1 for the lining of dental cavities and exhibits good water and acid stability. The materials of Example 1 were compared with this material for compressive strength as follows. Separate samples of each material were formed into 6 mm. diameter by 12 mm. length cylinders and allowed to harden. Each was then subjected to 37°C distilled water

immersion as described in Example 2. Each was then tested on an Instron™ testing machine for compressive strength. The neopentyl diester-containing material of Example 1 exhibited strengths in excess of 20 mega Pascals, MPa (approximately 23.5 MPa, $\delta$= 1.0, n = 4). The material of this Example had a strength of about 9.4 MPa ($\delta$= .96, n = 5).

<u>WHAT IS CLAIMED IS</u>:

1. A dental lining composition comprising:

metal oxide or hydroxide, and

an amount sufficient to impart substantial hydrophobicity to said composition of fluorinated polymeric hydrocarbon.

2. The composition of claim 1 wherein said fluorinated polymeric hydrocarbon is polytetrafluoroethylene.

3. The composition of claim 1 wherein said metal oxide or hydroxide comprises calcium hydroxide.

4. The composition of claim 1 further comprising an amount sufficient to impart radiopacity to said composition when employed as a dental lining of inorganic, radiopaque material.

5. A dental lining composition comprising:

metal oxide or hydroxide,

diester of salicylic acid specie and a compound having the formula

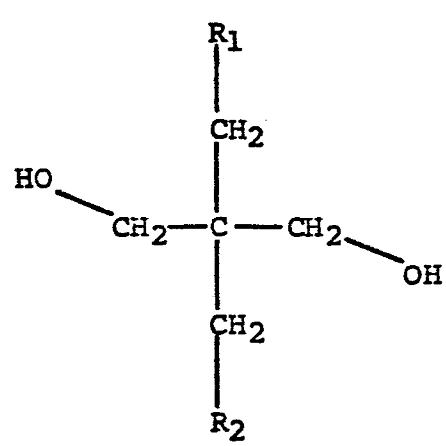

wherein $R_1$ and $R_2$ may be the same or different and are H or alkyl groups having from 1 to about 3 carbon atoms, and

an amount sufficient to impart substantial hydrophobicity to said composition of fluorinated polymeric hydrocarbon.

6. The composition of claim 5 wherein said fluorinated polymeric hydrocarbon is polytetrafluoroethylene.

7. The composition of claim 5 wherein said metal oxide or hydroxide comprises calcium hydroxide.

8. The composition of claim 5 further comprising an amount sufficient to impart radiopacity to said composition when employed as a dental lining of inorganic, radiopaque material.

9. The composition of claim 5 wherein said diester is neopentyl disalicylate.

10. The composition of claim 5 wherein said diester has been heated with aqueous inorganic acid for a period of time sufficient to avoid agglomeration of said diester.

11. A method for repairing a tooth comprising applying to a tooth cavity a dental cavity lining composition comprising:

metal oxide, and

an amount sufficient to impart substantial hydrophobicity to said composition of fluorinated polymeric hydrocarbon, and

filling said cavity with dental restorative.

12. The method of claim 11 wherein said fluorinated polymeric hydrocarbon is polytetrafluoroethylene.

13. The method of claim 11 wherein said metal oxide or hydroxide comprises calcium hydroxide.

14. The method of claim 11 further comprising an amount sufficient to impart radiopacity to said composition when employed as a dental lining of inorganic, radiopaque material.

15. The method of claim 11 wherein said composition further comprises diester of salicylic acid specie and a compound having the formula

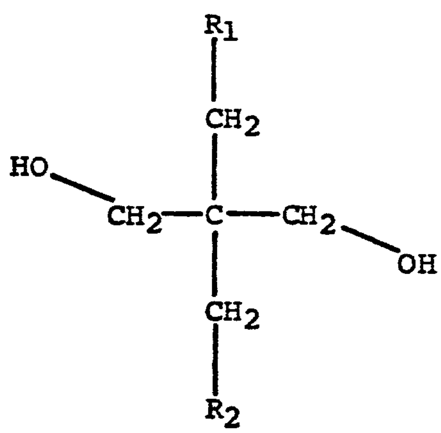

wherein $R_1$ and $R_2$ may be the same or different and are H or alkyl groups having from 1 to about 3 carbon atoms.

16. The method of claim 11 wherein said composition further comprises neopentyl disalicylate.

17. A diester of a salicylic acid species and a compound having the formula:

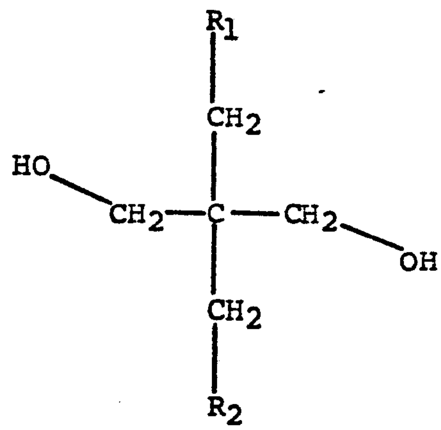

wherein $R_1$ and $R_2$ may be the same or different and are H or alkyl groups having from 1 to about 3 carbon atoms.

18. The diester of claim 17 consisting of neopentyl disalicylate.

19. The diester of claim 17 having been treated with an amount of an aqueous solution of an inorganic acid sufficient to diminish agglomeration of said diester.